# EUROPEAN PATENT SPECIFICATION

(11) **EP 2 046 339 B1**
(45) Date of publication and mention of the grant of the patent: **06.11.2013**
(21) Application number: 07786527.7
(22) Date of filing: 02.08.2007
(51) Int. Cl.: A61K 31/5513, A61K 31/47, A61K 31/435, A61K 31/4409, A61K 31/415, A61K 31/49, A61K 31/35, A61K 31/335, A61K 31/4709, A61P 33/06, A61P 33/00

(54) **RHO/ROCK/P13/AKT KINASE INHIBITORS FOR THE TREATMENT OF DISEASES ASSOCIATED WITH PROTOZOAN PARASITES**
RHO/ROCK/P13/AKT-KINASE-HEMMER ZUR BEHANDLUNG VON ERKRANKUNGEN IM ZUSAMMENHANG MIT PROTOZOEN-PARASITEN
INHIBITEURS DE RHO/ROCK/P13/AKT KINASE POUR LE TRAITEMENT DE MALADIES ASSOCIÉES AUX PARASITES PROTOZOAIRES

(30) Priority: 03.08.2006 EP 06291263
(43) Date of publication of application: 15.04.2009
(73) Proprietor: Université Pierre et Marie Curie (Paris VI), 75005 Paris (FR)
(72) Inventor: MAZIER, Dominique, 75010 Paris (FR); TAOUFIQ, Zacharie, 59700 Marcq-en-Baroeul (FR); CICERON, Liliane, 91340 Ollainville (FR); PINO, Paco, 1203 Genève (CH)
(74) Representative: Jacobson, Claude
(86) International application number: PCT/EP2007/006857
(87) International publication number: WO 2008/015001

(56) References cited:
- EP-A- 1 295 607
- EP-A- 1 550 660
- EP-A1- 1 256 574
- EP-A1- 1 270 570
- WO-A-2004/096222
- WO-A-2005/051392
- GODBOLD GENE D ET AL: "Cell killing by the human parasite Entamoeba histolytica is inhibited by the Rho-inactivating C3 exoenzyme" MOLECULAR AND BIOCHEMICAL PARASITOLOGY, vol. 108, no. 1, 30 April 2000 (2000-04-30), pages 147-151, XP002410914 ISSN: 0166-6851
- KHAN NAVEED AHMED: "Acanthamoeba: biology and increasing importance in human health" FEMS MICROBIOLOGY REVIEWS, vol. 30, no. 4, July 2006 (2006-07), pages 564-595, XP002410915 ISSN: 0168-6445

## Description

The present invention relates to pharmaceutical compositions for the treatment of protozoan parasitic diseases.

Malaria and African trypanosomiasis (sleeping sickness) represent the two major tropical vector-transmitted protozoan infections ¹⁷. Malaria, for instance, remains a major threat to public health with 3.2 billion people living in 107 countries currently at risk. Each year 300-500 millions clinical cases are reported and more than a million people die from severe complications associated with a *Plasmodium falciparum* infection (severe malaria) ¹, such as multi-organ failure (kidney, liver, lung, spleen) or an acute neurological syndrome called cerebral malaria (CM).

Cerebral malaria mostly affects young children and pregnant women in subsaharan Africa, whereas multi-organ failure is seen mainly in adults in South East Asia. Severe malaria cases may then progress rapidly, often within 24 hours, to coma and death if left untreated. Although CM has been extensively studied, its pathogenesis remains unraveled and there is no way to predict the onset of the disease. Moreover, the succession of events leading to cerebral complications and seizures is not well established. CM is diagnosed in patients living in malaria endemic areas or having recently traveled in such regions, showing acute neurological symptoms (from impaired consciousness to convulsions or coma) and positive peripheral blood smears for *P. falciparum* malaria parasites, when other causes for encephalopathy have been excluded ².

So far, emergency treatment for severe malaria has mainly consisted of intravenous administration of quinine, but research and clinical trials are currently performed on using other antimalarial drugs such as artemisinin and derivatives ³⁻⁵. Despite the high efficiency of those molecules in clearing the blood from parasites, 15-20% of mortality is still observed and several cases of delayed cerebellar ataxia or other cognitive neurological sequelae have been reported in treated patients after coma recovery ⁶⁻⁸. Thus, the current severe malaria treatments, which mainly rely on a direct anti-parasitic strategy, do not appear to be sufficient and other additional approaches are needed to improve the outcome of the disease.

Members of the Rho family of small GTPases are known to play a pivotal role in signal transmission of various membrane receptors. They act as molecular switches that are active when GTP-bound and inactive when GDP-bound, and are the first intermediates of intracellular signaling cascades with downstream effector Rho-kinase (ROCK) ¹⁰. Rho-kinase inhibitors (ROCKI) are currently investigated for vascular and neuroprotective therapy. Fasudil is a Rho-kinase inhibitor available for clinical use. It has been launched and widely used in Japan since 1995 against cerebral vasospasms in patients after subarachnoid hemorrhage and has lately shown to be safe and efficient in phase 2 clinical trials for patients with stable angina ¹⁶ or cerebral ischemic stroke ¹¹.

An object of the present invention is to provide treatments for pathologies associated with a parasitic infection.

The article Godbold and Mann (2000) Mol. Biochem. Parasitol. 108:147-151 describes the inhibitory activity of a truncated form of exoenzyme C3, devoid of its secretion signal and produced inside *Entamoeba histolytica* cells, on the parasite Rho protein.

The article Khan (2006) FEMS Microbiol. Rev. 30:564-595 shows that the Y27632 inhibitor of Rho kinase partially blocks *Acanthamoeba* phagocytosis.

The international application WO 2005/05392 describes compounds that disturb the GTP-binding activity of GTPases of the Ras family, in particular of the Rac GTPases of the Rho family which do not target Rho kinases, and the use thereof for treating various diseases including malaria and trypanosomiasis.

The international application WO 2004/096222 discloses the use of fasudil for treating and/or preventing hypopigmentary diseases.

### Summary of the invention

The present invention arises from the unexpected finding, by the Inventors, that Fasudil is liable to prevent *Plasmodium falciparum*-induced cell death, endothelium disruption, and endothelial activation.

Thus, the present application describes a method for preventing and/or treating pathologies associated with an infection by a protozoan parasite in an individual, comprising administering a therapeutically effective amount of a Rho/ROCK/PI3K/Akt pathway modulator to said individual.

An embodiment of the above defined method comprises further administering at least one compound having anti-parasitic activity.

The present application also describes the use of a Rho/ROCK/PI3K/Akt pathway modulator for the manufacture of a medicament intended for the prevention and/or the treatment of pathologies associated with an infection by a protozoan parasite.

In an embodiment of the above defined use, the medicament further comprises at least one compound having anti-parasitic activity.

The present application also discloses a pharmaceutical composition comprising as active substances:
- at least one Rho/ROCK/PI3K/Akt pathway modulator, and
- at least one compound having anti-parasitic activity,
in association with a pharmaceutically acceptable carrier.

The present application also discloses products containing:
- at least one Rho/ROCK/PI3K/Akt pathway modulator, and
- at least one compound having anti-parasitic activity,
as a combined preparation for simultaneous, separate or sequential use for the prevention or the treatment of pathologies associated with an infection by a protozoan parasite.

The present invention relates to a Rho kinase inhibitor for use as a medicament in the prevention and/or the treatment of pathologies associated with an infection by a protozoan parasite, wherein the Rho kinase inhibitor inhibits the Rho kinase of the cells of the individual who is at risk of being infected or is infected by a protozoan parasite.

### Detailed description of the invention

### Rho/ROCKlPI3K/Akt pathway modulator

As intended herein, the Rho/ROCK/PI3K/Akt pathway comprises a Rho GTP-binding protein, preferably RhoA; a Rho kinase (ROCK), preferably a RhoA kinase; phosphatidyl insositol 3 kinase (PI3K); Akt kinase; the cellular targets of Akt kinase, such as endothelial nitric oxide synthase (eNOS); and the cellular membrane components which are liable to activate the Rho protein by interacting with it, such as membrane-borne adhesion molecules. This pathway is notably defined in Wolfrum et al. (2004) Arterioscler. Thromb. Vasc. Biol. 24:1842-1847. The Inventors have shown that this pathway could be activated in cells, in particular endothelial cells, as a consequence of parasite cytoadherence. More precisely, adherence of parasitized cells or of parasites at the surface of uninfected target-cells of an infected organism induces, in these target cells, the activation of Rho through membrane borne adhesion molecules; activated Rho then activates a Rho-kinase which, among several other targets, inhibits PI3K and Akt kinase. In endothelial cells adhered by infected erythrocytes for example, Akt kinase inhibition can in turn be responsible for eNOS inhibition, which in particular relieves the negative control eNOS exerts on apoptosis, thereby inducing apoptosis of the endothelial adhered cells and endothelium disruption.

As intended herein a "Rho/ROCK/PI3K/Akt pathway modulator" relates to a compound which is liable to modulate the activity of the Rho/ROCK/PI3K/Akt pathway in a way such that the outcome of the pathway is the opposite of what normally occurs upon activation of the Rho protein. As is apparent, the modulator aims at modulating the Rho/ROCK/PI3K/Akt pathway of the cells of the individual who is at risk of being infected or is infected by a protozoan parasite. In particular the Rho/ROCK/PI3K/Akt pathway modulator is liable (i) to inhibit at least one of Rho and Rho kinase, and/or (ii) to activate at least one of PI3K, Akt kinase and eNOS. Preferably, the Rho/ROCK/PI3K/Akt pathway modulator acts by binding to its target protein (e.g. Rho, Rho kinase, PI3K, Akt kinase or eNOS). Such Rho/ROCK/PI3K/Akt pathway modulators are useful, in particular to prevent the negative outcome of parasitic cytoadherence in infected organisms.

Preferably, the Rho/ROCK/PI3K/Akt pathway modulator is a Rho kinase inhibitor (ROCKI). ROCKIs are notably described in Tamura et al. Development of specific Rho-kinase inhibitors and their clinical application Biochim. Biophys. Acta 2005;1754:245-252. Other ROCKIs are also described in EP 1 256 574, EP 1 270 570, and EP 1 550 660.

More preferably, the Rho/ROCK/PI3K/Akt pathway modulator is a ROCKI selected from the group consisting of the compounds of the following formulae: 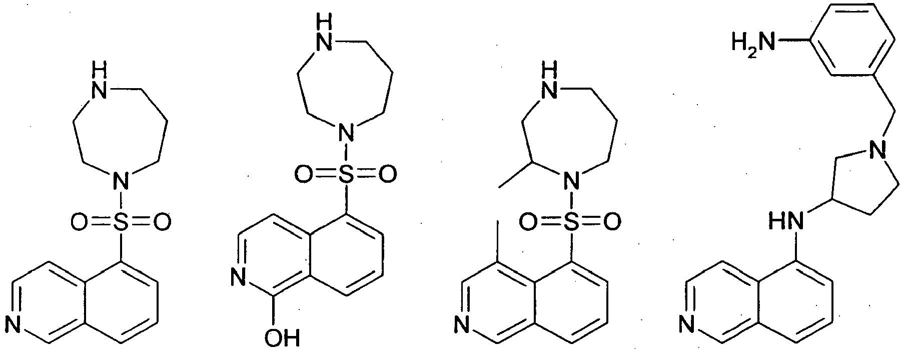 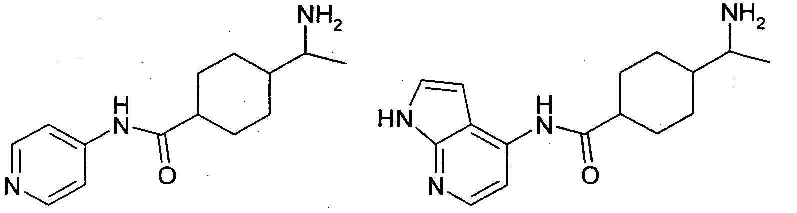 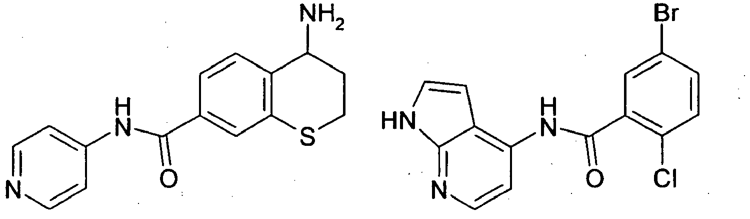 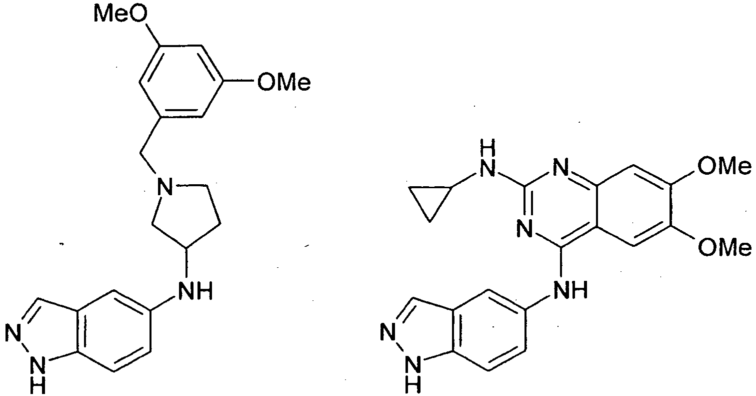 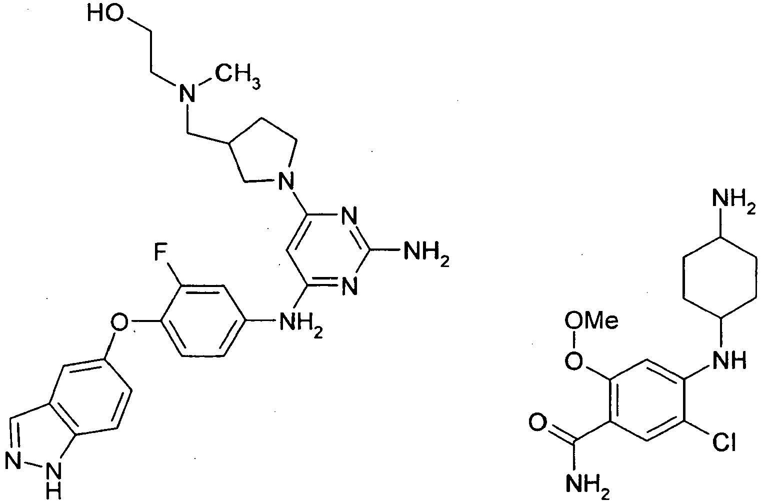 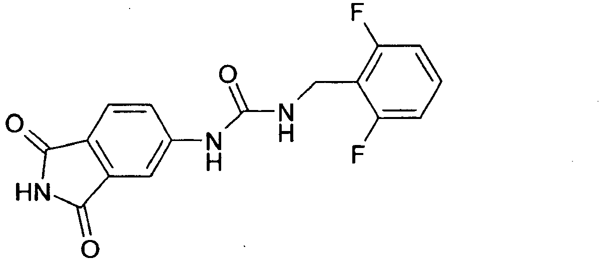 or pharmaceutically acceptable salts thereof.

Most preferably, the Rho/ROCK/PI3K/Akt pathway modulator is a ROCKI selected from the compound of the following formulae: 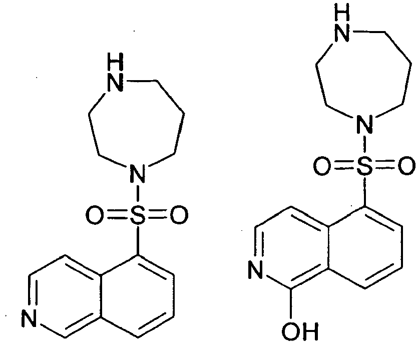 or pharmaceutically acceptable salts thereof.

These latter two compounds are respectively named Fasudil (also known as HA-1077 or AT877) and Hydroxyfasudil (also known as H-4413). Hydroxyfasudil is an active metabolite of Fasudil.

As intended herein, pharmaceutically acceptable salts relate to the relatively non-toxic, inorganic and organic acid addition salts, and base addition salts, of the above mentioned compounds. Exemplary salts include the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, sulphamates, malonates, salicylates, propionates, methylene-bis-b-hydroxynaphthoates, gentisates, isethionates, di-p-toluoyltartrates, methane-sulphonates, ethanesulphonates, benzenesulphonates, p-toluenesulphonates, cyclohexylsulphamates, quinateslaurylsulphonate, amine, and metal (e.g. sodium, potassium, calcium, barium, zinc, magnesium, or aluminium) salts, and the like (see, for example, Berge et al. (1977) «Pharmaceutical Salts» J. Pharm. Sci. 66:1-19).

The hydrochloride salt of Fasudil is preferred.

### Pathologies

As intended herein, "pathologies associated with an infection by a protozoan parasite" relate to the disorders, the diseases or the syndromes which are directly or indirectly a consequence of an infection by a protozoan parasite. The pathologies according to the invention preferably arise, partially or totally, from parasite-induced apoptosis, cellular activation, in particular endothelial activation, and/or endothelium disruption, which themselves can in particular arise from parasitic cytoadherence.

Preferably, the protozoan parasite is selected from the group consisting of *Trypanosoma spp* and *Plasmodium spp.* In particular, the protozoan parasite can be selected from the group consisting of *Plasmodium falciparum* and *Trypanosoma brucei spp.,* such as *Trypanosoma brucei brucei, Trypanosoma brucei gambiense,* and *Trypanosoma brucei rhodesiense.*

The pathologies are preferably selected from the group consisting of malaria, and African trypanosomiasis (sleeping sickness).

More preferably, the pathologies are selected from the group consisting of severe malaria, cerebral malaria, multi-organ failure syndrome, and advanced-stage African trypanosomiasis.

### Compound having anti-parasitic activity

As intended herein, a "compound having anti-parasitic activity" relates to any compound which is used in the course of the treatment of infections by a protozoan parasite or their pathological consequences. The compound having anti-parasitic activity preferably relates to a compound used for decreasing the parasite load in an infected organism.

Rho/ROCK/PI3K/Akt pathway modulators are useful as adjunctive treatment in parasitic diseases. As such, the association of a Rho/ROCK/PI3K/Akt pathway modulator and of a compound having anti-parasitic activity is advantageous since it destroys the parasite itself, while preventing and/or treating consequences of parasitic infection, such as cellular activation, in particular endothelial activation, endothelium disruption, and apoptosis, which are induced by parasitic cytoadherence.

Preferably, the compound having anti-parasitic activity is a compound having anti-malarial activity.

Preferably, the compound having anti-malarial activity is selected in the group consisting of Quinine, Quinidine, Quinine-doxycycline, Artemether, Artemotil, Artesunate, Arteether, Méfloquine, Amodiaquine, Dihydroartémisinine, Pipéraquine, Halofantrine, Atovaquone, Chloroquine, Dapsone, Doxycycline, a Cycline, Lumefanthrine, Proguanil, Pyrimethamine, Pyronaridine, Sulfadoxine, Diamidine, Ferroquine, Fluoroquinolone, Fosmidomycine, Tafenoquine, and Trioxaquine.

### Administration

The Rho/ROCK/PI3K/Akt pathway modulator, the compound having anti-parasitic activity, medicament, the pharmaceutical composition, or the products described in the application can be administered by the oral, intradermal, intraveinous, intramuscular or intrarectal route.

The Rho/ROCK/PI3K/Akt pathway modulator, the compound having anti-parasitic activity, medicament, the pharmaceutical composition, or the products described in the application can be administered in the form of solutions, suspensions, tablets, or capsules.

The choice of the "pharmaceutically acceptable carrier" can be routinely determined by the man skilled in the art. It is generally determined in accordance with the solubility and chemical properties of the active substance. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The dose at which the Rho/ROCK/PI3K/Akt pathway modulator, the compound having anti-parasitic activity, the medicament, the pharmaceutical composition, or the products described in the application can be administered to an individual in need thereof can be routinely determined by the man skilled in the art. In particular, the compound having anti-parasitic activity can be administered at the same dose than when it is not used in association with the Rho/ROCK/PI3K/Akt pathway modulator described in the application.

By way of example Fasudil can be administered at a dosage of from 50 mg/ day to 500 mg/day, in particular at 240 mg/day, with unit doses of from 50 mg to 500 mg, in particular 80 mg.

### Description of the figures

**Figure 1**

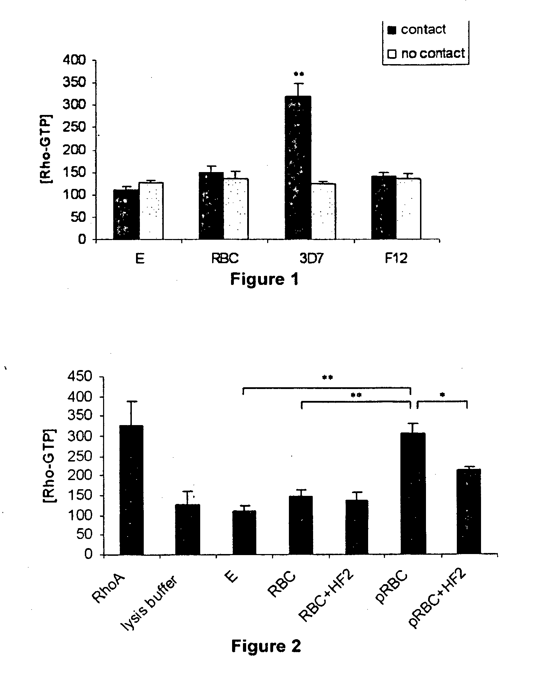

   Figure 1 represents the cytoplasmic concentration of activated RhoA ([Rho-GTP], vertical axis, arbitrary units) for an endothelial cell extract from:
   (E) control endothelial cells under contact (black column) or no contact (white column) conditions;
   (RBC) endothelial cells contacted (black column) or not (white column) by control red blood cells;
   (3D7) endothelial cells contacted (black column) or not (white column) by 3D7 clone parasitized red blood cells;
   (F12) endothelial cells contacted (black column) or not (white column) by F12 clone parasitized red blood cells.
   Results were obtained after measurement of optical density at 490nm of cell extracts which stands for [Rho-GTP]. Data represent the mean +/- SD of 3 independent experiments. ** P<0.01.
**Figure 2**
   Figure 2 represents the cytoplasmic concentration of activated RhoA ([Rho-GTP], vertical axis, arbitrary units) for:
   **(RhoA)** a positive control consisting of purified RhoA protein provided in the G-LISA™ kit;
   **(lysis buffer)** the G-LISA™ cell extraction buffer;
   **(E)** an endothelial cell extract from control endothelial cells;
   **(RBC)** an endothelial cell extract from endothelial cells contacted by control red blood cells;
   **(RBC+HF2)** an endothelial cell extract from endothelial cells contacted by control red blood cells and with Fasudil at 30µM during 24 hours at 37°C
   **(pRBC)** an endothelial cell extract from endothelial cells contacted by parasitized red blood cells;
   **(pRBC+HF2)** an endothelial cell extract from endothelial cells contacted by parasitized red blood cells and with Fasudil at 30µM during 24 hours at 37°C;
   Results were obtained after measurement of optic density at 490nm of cell extracts which stands for [Rho-GTP]. Data represent the mean +/- SD of 3 independant experiments. **P*<0.05; ** *P*<0.01.
**Figure 3**

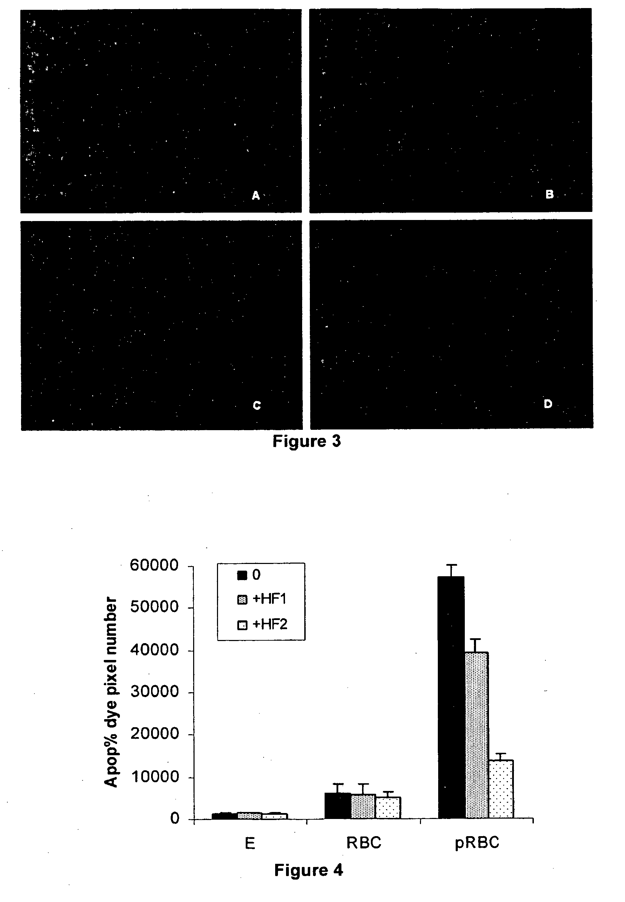

   Figure 3 represents inverted phase contrast microscope photographs of **(panel A)** non-exposed endothelial cells; **(panel B)** endothelial cells exposed to control red blood cells; **(panel** C) endothelial cells exposed to parasitized red blood cells, (**panel D)** endothelial cells exposed to parasitized red blood cells and to Fasudil at 30µM; in the presence of Apoppercentage™ purple dye which is uptaken by apoptotic cells. Arrows mark purple dye uptake.
**Figure 4**
   Figure 4 represents the number of purple dye pixels (Apop%, vertical axis) for inverted phase contrast microscope photographs as described in Figure 3, for (E) non-exposed endothelial cells, **(RBC)** endothelial cells exposed to control red blood cells, **(pRBC)** endothelial cells exposed to parasitized red blood cells, and incubated with 0 µM (black columns), 1µM (gray columns) or 30µM (dotted columns) Fasudil.
   Pixel numbers were determined using Adobe® Photoshop®. Data represent the mean +/- SD of pixel number from 4 photographs of representative field of 3 independent experiments.
**Figure 5**

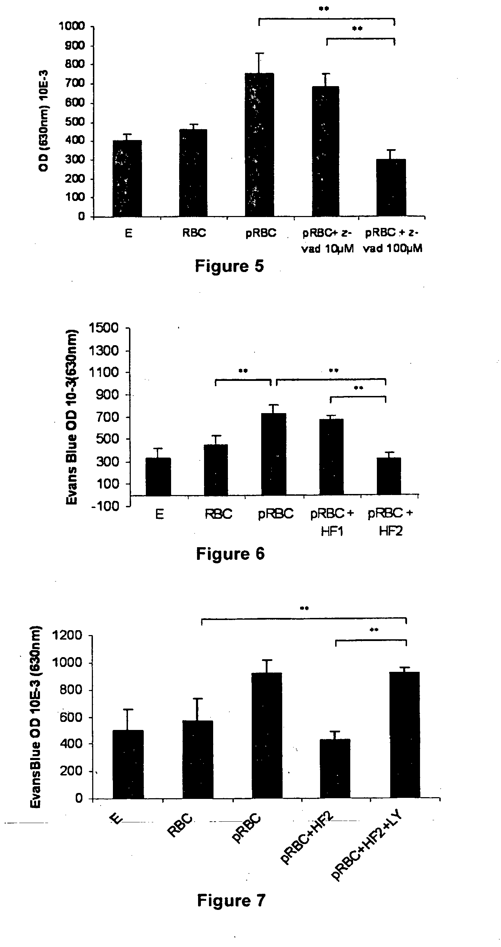

   Figure 5 represents endothelial barrier integrity (optical density (OD) of the Evans Blue marker at 630 nm, vertical axis, x10⁻³) for **(E)** non-exposed endothelial cells, **(RBC)** endothelial cells exposed to control red blood cells, **(pRBC)** endothelial cells exposed to parasitized red blood cells, **(pRBC+z-vad 10µM)** endothelial cells exposed to parasitized red blood cells and incubated with the broad range caspase inhibitor z-vad at 10µM, **(pRBC+z-vad 100µM)** endothelial cells exposed to parasitized red blood cells and incubated with z-vad at 100µM.
   Data represent the mean +/- SD of 10⁻³ optic density values from 3 independent experiments and 6 replicates for each condition. ** P<0.01.
**Figure 6**
   Figure 6 represents endothelial barrier integrity (optical density (OD) of the Evans Blue marker at 630 nm, vertical axis, x10⁻³) for **(E)** non-exposed endothelial cells, **(RBC)** endothelial cells exposed to control red blood cells, **(pRBC)** endothelial cells exposed to parasitized red blood cells, **(pRBC+HF1)** endothelial cells exposed to parasitized red blood cells and subsequently treated with 1µM Fasudil during 20 hours, **(pRBC+HF2)** endothelial cells exposed to parasitized red blood cells and subsequently treated with 30µM Fasudil during 20 hours.
   Data represent the mean +/- SD of 10⁻³ optic density values from 3 independent experiments and 6 replicates per each condition. ** *P*<0.01.
**Figure 7**
   Figure 7 represents endothelial barrier integrity (optical density (OD) of the Evans Blue marker at 630 nm, vertical axis, x10⁻³) for **(E)** non-exposed endothelial cells, **(RBC)** endothelial cells exposed to control red blood cells, **(pRBC)** endothelial cells exposed to parasitized red blood cells, **(pRBC+HF2)** endothelial cells exposed to parasitized red blood cells and subsequently treated with 30µM Fasudil during 20 hours, **(pRBC+HF2+LY)** endothelial cells exposed to parasitized red blood cells and subsequently treated with 30µM Fasudil and 15 µM PI3K inhibitor LY294002 during 20 hours.
   Data represent the mean +/- SD of 10⁻³ optic density values from 3 independent experiments and 3 replicates per each condition. ** P<0.01.
**Figure 8**

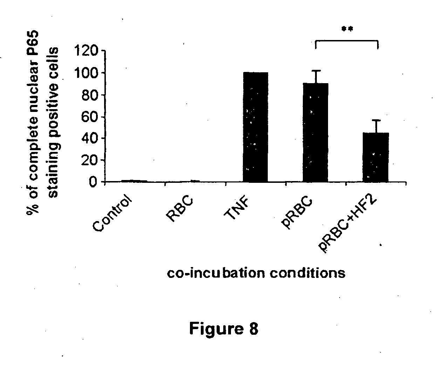

   Figure 8 represents the number of activated HLEC (cells with complete but not partial migration nuclear NF-kappaB P65 subunit staining) either non-exposed (control) or exposed to non-infected red blood cells **(RBC),** TNFα, parasitized red blood cells **(pRBC)** and parasitized red blood cells + Fasudil at 30 µM **(pRBC+HF2)** was counted. Data represent the mean +/- SD of % of positive nuclear P65 cells. ** *P*<0.005.

### EXAMPLES

### Materials and Methods:

### Culture of human lung endothelial cells (HLEC)

Primary endothelial cells (HLEC) were isolated from human lung after enzymatic digestion and selected using a continuous gradient and immunomagnetic purification technique as previously described ¹². Endothelial cells of ninth to twelfth passages derived from one batch were used for the experiments. Before use, cells were verified for their expression of ICAM-1, CD36, Von Willebrand factor, VCAM-1, CD31, E/P-selectin and CSA as previously described. HLEC were raised in M199 medium (Gibco), supplemented with 10µg/ml endothelial cell growth supplement (ECGS) (Upstate, Lake Placid, NY) and 10% of fetal calf serum (Biowest, South America origin), at 37°C with 5% CO₂, using 8 chambers lab-tek (Nalge Nunc International, Napervil, I11), 6-well plate (Becton Dickinson), or Transwell insert supports (Corning Incorporated).

### Plasmodium falciparum culture

*P. falciparum* 3D7 ¹⁴ and F12 ⁹ clones were used for our experiments. *P. falciparum* infected red blood cells (pRBCs) were maintained in culture according to Trager and Jensen's technique in a suspension of red blood cells (RBC) in RPMI (Gibco) supplemented with 8.3 g.l⁻¹ of Hepes, 2.1 g.l⁻¹ of NaHCO, 0.1 mg.ml⁻¹ of gentamycin and 2g.l⁻¹ of dextrose and 0.4% of Albumax II (Gibco, Invitrogen Corporation) ¹³. The 3D7 clone was characterized for adhesion phenotype as previously described ¹⁴ and adheres to HLEC on ICAM-1 and CD36 at 30% and 45% respectively. Its derivative F12 clone was used for its poor adhesive property ⁹ (1 pRBC/20 HLEC at maximum, compared with the 3D7 clone, for which >1 pRBC/HLEC is usually observed). For each experiment, parasite cultures were enriched in mature forms by Plasmagel floating ¹⁵. Briefly, erythrocytes were harvested from 5 to 10% parasitized cultures and centrifuged 5 minutes at 2000 rpm. Cells were resuspended in Plasmion® (fresenius kabi, France) and incubated during 25 minutes at 37°C. The upper fraction containing mature trophozoites and schizonts was then collected and washed 3 times in RPMI. Parasitized red blood cell suspensions were then adjusted to 1% of hematocrit and 50% of parasitemia.

### Reagents

Lyophilized Rho Kinase inhibitor Fasudil (HA-1077) was purchased from Biaffin (Kassel, Germany) and reconstituted in PBS. Fasudil was used at 1µM (HF1), 30µM (HF2) or 100µM (HF3). Phosphatidylinositol 3-kinase inhibitor LY294002 was purchased from Calbiochem and solubilized in ethanol in a 15mM stock solution before use at 15µM (LY). Broad range inhibitor of caspases Z-VAD-fmk was purchased from Sigma. All reagents were stored at -20°C.

### Endothelial barrier integrity assay

In order to measure the effect of treatments on pre-exposed pRBCs endothelial cells, we developed a method based on Evans Blue diffusion through endothelial monolayer. Briefly HLEC were raised on Transwell Permeable supports (polyester membrane, 3µm pore size, 6.5mm diameter, Corning Incorporated *Life Sciences)* until confluence in M199 medium. Mature forms of 3D7 *P. falciparum* clone were then harvested from culture and adjusted at 1% of hematocrit and 50% of parasitemia before 4 hours co-incubation at 37°C 5% CO₂. After pRBC exposure, endothelial cell monolayers were treated 20 hours with Fasudil (1µM or 30µM). Similar incubations with non infected erythrocytes or endothelial cell alone were taken as controls. Transwell compartments were then washed 3 times with RPMI-based adhesion medium. Transwell inserts were transferred to a new plate filled with PBS; a solution of Evans Blue (ICN Biomedicals Inc.) at 0.5mg/mL was then added in the upper compartments and incubated for 5 minutes at 37°C 5% CO₂. Lower compartments were finally collected for optical density analysis at 630nm of diffused Evans Blue, using a standard microtiter plate reader (Bio-Tek™ EL311SX, Instruments Inc).

### Quantitative apoptosis assay

Endothelial apoptosis was quantitatively assessed using APOPercentage™ dye purchased from Biocolor Ltd. (Belfast, Northern Ireland). The assay uses a purple-red colour dye that is selectively incorporated into apoptotic but not necrotic cells. Unilateral uptake of the dye occurs through phosphatidylserines of the outer membrane of cells that undergo apoptosis. Endothelial cells were cultured in 6-well plates until confluence before exposure to *P. falciparum* infected erythrocytes during 24 hours. HLEC were incubated for 1 hour with the Apopercentage dye (1/20) before co-incubation time is reached and delicately washed 3 times with PBS. Apoptosis was then quantified by Analytical Digital Photomicroscopy (ADP): for each experiment 4 representative fields of each well of cell cultures were photographed using inverted phase contrast microscope coupled with a digital camera. Images were then transferred to a computer and the level of apoptosis was expressed as a red pixel number counted by Adobe^{®} Photoshop^{®}.

### RhoA/Rho-Kinase pathway activation assay

Activity of RhoA and its downstream effector Rho-kinase was assessed using G-LISA™ RhoA Activation Assay Biochem Kit purchased from Cytoskeleton (Denver, CO). The principle of the assay is to determine the amount of active GTP-bound RhoA extracted from cultured cells with a Rho-GTP-binding protein coated 96-well plate and final detection with immunoreagents. Briefly, HLEC were cultured in 6-well plates until 50-70% confluence as recommended by the manufacturer and exposed to *P. falciparum* infected erythrocytes or non infected erythrocytes at 1% of hematocrit and 50% of parasitemia. When used, Fasudil was added concomitantly with parasites at 30µM. After 24 hours of incubation cells were washed and ice-cold lysed in phosphate buffer saline containing protease inhibitors. Cell lysates were then clarified by centrifugation at 4°C 10,000 rpm for 2 min before measurement of protein concentrations. Cell extracts were equalized with lysis buffer to give identical protein concentrations before incubation in Rho-GTP affinity plate for 30 min at 4°C. Antibody detection reagents (primary anti-RhoA antibody, secondary antibody, HRP detection reagents) provided in G-LISA™ kit were added and signal was developed with luminometry analysis (490nm). In no-contact assay, HLEC were subcultured in the lower compartment of Transwell (polyester membrane of 0.4µM pores) (Corning Incorporated *Life Sciences)* until confluence. PRBC and control RBC were then added in the upper compartment and incubated 24 hours at 37°C before performing G-LISA™ test as described above.

### Statistical analysis

Each experiment was carried out three times independently and data collected from each co-culture conditions are expressed as mean ± SE. Results were analyzed for statistical significance using the one-way ANOVA followed by the Tukey multiple comparison test. A value of *p*< 0.05 was considered significant.

### Example 1

### Fasudil can prevent RhoA/Rho-kinase pathway activation induced by P. falciparum contact in human endothelial cells

The Inventors first established that *P. falciparum* is able to trigger the RhoA/Rho-kinase pathway in endothelial cells.

Briefly, RhoA activity in HLEC co-incubated during 24 hours with *P. falciparum* infected red blood cells (pRBC) or non infected red blood cells (RBC) was measured using the G-LISA™ RhoA activation kit (Cytoskeleton, Denver CO) as described in the Materials and Methods section.

As shown in **Fig. 1****,** HLEC co-incubated during 24 hours with infected erythrocytes showed significant increase in cellular activated RhoA concentrations compared to HLEC exposed to non infected erythrocytes (p<0.01) or non-exposed HLEC (p<0.01).

Furthermore the Inventors showed that a physical contact between HLEC and infected erythrocytes (pRBC) is required for RhoA activation.

Briefly, HLEC were co-incubated in transwell with pRBC or control RBC in a 'no-contact' assay to avoid cytoadherence (Materials and Methods section). In this condition, compartments were separated by a porous membrane (0.4µm pores) which prevented erythrocytes to adhere to endothelial cells but did not prevent passage of macromolecules and media. Co-incubation with pRBC in no-contact assay showed a similar activated RhoA concentration to basal levels.

This was further confirmed using cytoadhesive-less *P. falciparum* F12 clone instead of the *P. falciparum* 3D7 clone for preparing pRBCs. F12 could not induce the RhoA/Rho-kinase pathway.

The Inventors then showed that a known RhoA/Rho-kinase inhibitor, Fasudil, at 30µM in 24 hours pRBC-contact co-incubation was liable to induce a significant decrease in activated RhoA concentration compared to pRBC alone (p<0.05) **(****Fig. 2****).**

### Example 2

### Rho-Kinase inhibitor Fasudil decreases P. falciparum induced endothelial cell apoptosis

The Inventors then showed that Fasudil is liable to protect endothelial cells from P. *falciparum*-induced apoptosis.

Briefly, APOPercentage™ was used as a mean to quantify induced apoptosis and the effect of Fasudil on cell survival during pRBC/HLEC co-incubations by quantitatively assessing the release of purple red dye by apoptotic HLEC using Analytical Digital Photomicroscopy (ADP). HLEC were either non-exposed **(****Fig. 3****, panel A),** co-incubated with RBC **(****Fig. 3****, panel B),** or co-incubated with pRBC alone **(****Fig. 3****, panel C),** with fasudil at 1µM **(****Fig. 3****, panel D)** or with fasudil at 30µM **(****Fig. 4****).**

Thus, the Inventors observed that pRBC were able to trigger HLEC apoptosis as compared to RBC (9.5 fold). However, when cells are treated with 1µM of Fasudil, a decrease in apoptosis of pRBC-exposed HLEC compared to non treated cells is observed (1.4 fold decrease). This decrease in apoptosis is strengthened (4.1 fold decrease) when cells are treated with a higher concentration of Fasufil (30µM).

### Example 3

### Reversion of P. falciparum induced endothelial permeability with a Rho-kinase inhibitor

The Inventors have shown that *P. falciparum*, likely because of cellular apoptosis, may have drastic effects on endothelium permeability leading to a loss of barrier integrity.

Briefly, *P. falciparum* cytoadherence effect on endothelial barrier function was assessed by culturing HLEC in Transwell inserts until confluence before exposing them 4 hours to mature forms of pRBC as above. The quantity of Evans Blue collected in lower compartments was markedly higher in pRBC/HLEC co-incubation conditions than in control RBC/HLEC (p<0.01) or non-exposed endothelial cell conditions (p<0.01). Evans Blue collected in those controls remains at equivalent levels and the slight increase observed in the case of RBC co-incubations is not significant. Furthermore, the quantity of Evans Blue in the lower compartment in pRBC/HLEC co-incubation conditions was significantly reduced in the presence of the broad-range caspase inhibitor z-vad **(****Fig**. **5****)**. Thus, the passage of Evans Blue was relatively prevented reflecting an improvement of monolayer permeability through apoptosis inhibition.

The Inventors then showed that Fasudil could prevent *P. falciparum* induced endothelial permeability.

Briefly, HLEC were cultured in Transwell inserts until confluence. The obtained cell monolayer was then exposed during 4 hours to pRBC adhesion and subsequently treated 20 hours with 1µM or 30µM of Fasudil **(****Fig. 6****)** and/or with 15µM of PI3K inhibitor LY294002 **(****Fig. 7****).** Endothelial barrier integrity was assessed as described above.

When the incubation was carried with HLEC, pBRC and Fasudil at 30µM the quantity of Evans Blue marker was found significantly lower than when incubation was carried with HLEC and pRBC (p<0.01) **(****Fig. 6****).** Besides, the effect of Fasudil could be reversed by LY294002, which shows that the effects of Fasudil derive, at least in part, from PI3K activation subsequent to Rho kinase inhibition **(****Fig. 7****).**

### Example 4

### Effect of Fasudil on P. falciparum induced NF-kappaB endothelial activation

Endothelial activation is believed to play a crucial role in *P. falciparum* malaria acute phase severity ¹⁸. *P. falciparum* cytoadherence is known to induce proinflammatory endothelial responses via signaling cascades implying NF-kappaB transcription factor ^{19, 20}. Upon those stimuli, NF-kappaB is phosphorylated on specific residues, becomes activated and translocates into the nucleus.

The inventors therefore tested whether Fasudil would also modulate *P*. *falciparum*-induced endothelial activation.

Briefly, HLEC were cultured in 8-chambers Labtek plates and exposed to pRBC during 6 hours at 1% of hematocrit and 50% of parasitemia. Rho-kinase inhibitor Fasudil was added concomitantly with pRBC at 30µM. TNFα at 20ng/ml was used as a positive control of NF-kappaB nuclear translocation. Cells were then fixed and permeabilized for immunostaining with primary anti-NF-kappaB P65 subunit antibody and secondary FITC-anti-mouse IgG antibody. Nuclei were stained with diamidino-phenylindole (DAPI). After immunostaining of NF-kappaB P65 subunit, results were analysed by fluorescence microscopy and the number of activated HLEC (cells with complete but not partial migration nuclear P65 staining) per each condition (n=4) was counted (Fig.8).

Results indicate that Fasudil significantly reduces *P. falciparum*-induced endothelial activation, as shown by the inhibition of *P. falciparum*-induced NF-kappaB nuclear translocation.

### References

1. UNICEF/WHO. World Malaria Report 2005. www.who.int/malaria 2005.
2. WHO DoCoTD. Severe falciparum malaria. Trans R Soc Trop Med Hyg 2000;94((Suppl 1)):1-90.
3. van Hensbroek MB, Onyiorah E, Jaffar S, et al. A trial of artemether or quinine in children with cerebral malaria. N Engl J Med 1996;335(2):69-75.
4. Aceng JR, Byarugaba JS, Tumwine JK. Rectal artemether versus intravenous quinine for the treatment of cerebral malaria in children in Uganda: randomised clinical trial. Bmj 2005;330(7487):334.
5. Ashley EA, White NJ. Artemisinin-based combinations. Curr Opin Infect Dis 2005;18(6):531-6.
6. Brewster DR, Kwiatkowski D, White NJ. Neurological sequelae of cerebral malaria in children. Lancet 1990;336(8722):1039-43.
7. White NJ. Not much progress in treatment of cerebral malaria. Lancet 1998;352(9128):594-5.
8. Idro R, Jenkins NE, Newton CR. Pathogenesis, clinical features, and neurological outcome of cerebral malaria. Lancet Neurol 2005;4(12):827-40.
9. Pino P, Vouldoukis I, Kolb JP, et al. Plasmodium falciparum--infected erythrocyte adhesion induces caspase activation and apoptosis in human endothelial cells. J Infect Dis 2003;187(8):1283-90.
10. Cernuda-Morollon E, Ridley AJ. Rho GTPases and leukocyte adhesion receptor expression and function in endothelial cells. Circ Res 2006;98(6):757-67.
11. Shibuya M, Hirai S, Seto M, Satoh S, Ohtomo E. Effects of fasudil in acute ischemic stroke: results of a prospective placebo-controlled double-blind trial. J Neurol Sci 2005;238(1-2):31-9.
12. Muanza K, Gay F, Behr C, Scherf A. Primary culture of human lung microvessel endothelial cells: a useful in vitro model for studying Plasmodium falciparum-infected erythrocyte cytoadherence. Res Immunol 1996;147(3):149-63.
13. Trager W, Jensen JB. Human malaria parasites in continuous culture. Science 1976;193(4254):673-5.
14. Ockenhouse CF, Klotz FW, Tandon NN, Jamieson GA. Sequestrin, a CD36 recognition protein on Plasmodium falciparum malaria-infected erythrocytes identified by anti-idiotype antibodies. Proc Natl Acad Sci U S A 1991;88(8):3175-9.
15. Goodyer ID, Johnson J, Eisenthal R, Hayes DJ. Purification of mature-stage Plasmodium falciparum by gelatine flotation. Ann Trop Med Parasitol 1994;88(2):209-11.
16. Vicari RM, Chaitman B, Keefe D, et al. Efficacy and safety of fasudil in patients with stable angina: a double-blind, placebo-controlled, phase 2 trial. J Am Coll Cardiol 2005;46(10):1803-11.
17. Bisser S., et al. Harbouring in the brain: A focus on immune evasion mechanisms and their deleterious effects in malaria and human African trypanosomiasis. Int. J. Parasitol. 2006;36:529-540.
18. Hollestelle, M.J., Donkor, C., Mantey, E.A., Chakravorty, S.J., Craig, A., Akoto, A.O., et al (2006) von Willebrand factor propeptide in malaria: evidence of acute endothelial cell activation. BrJ Haematol. 133: 562-569.
19. Schofield, L., Novakovic, S., Gerold, P., Schwarz, R.T., McConville, M.J. and Tachado, S.D. (1996) Glycosylphosphatidylinositol toxin of Plasmodium up-regulates intercellular adhesion molecule-1, vascular cell adhesion molecule-1, and E-selectin expression in vascular endothelial cells and increases leukocyte and parasite cytoadherence via tyrosine kinase-dependent signal transduction. J Immunol. 156: 1886-1896.
20. Tripathi, A.K., Sullivan, D.J. and Stins, M.F. (2006) Plasmodium falciparum-infected erythrocytes increase intercellular adhesion molecule 1 expression on brain endothelium through NF-kappaB. Infect Immun. 74: 3262-3270.

## Claims

1. A Rho kinase inhibitor for use as a medicament in the prevention and/or the treatment of pathologies associated with an infection by a protozoan parasite, wherein the Rho kinase inhibitor inhibits the Rho kinase of the cells of the individual who is at risk of being infected or is infected by a protozoan parasite.

2. The Rho kinase inhibitor for its use according to claim 1, wherein the protozoan parasite is selected from the group consisting of *Trypanosoma spp.* and *Plasmodium spp.*

3. The Rho kinase inhibitor for its use according to claim 1 or 2, wherein the pathologies are selected from the group consisting of malaria and African trypanosomiasis (sleeping sickness).

4. The Rho kinase inhibitor for its use according to any of claims 1 to 3, wherein the pathologies are selected from the group consisting of severe malaria, cerebral malaria, multi-organ failure syndrome, and advanced-stage of African trypanosomiasis.

5. The Rho kinase inhibitor for its use according to any of claims 1 to 4, which is selected from the group consisting of the compounds of the following formulae: 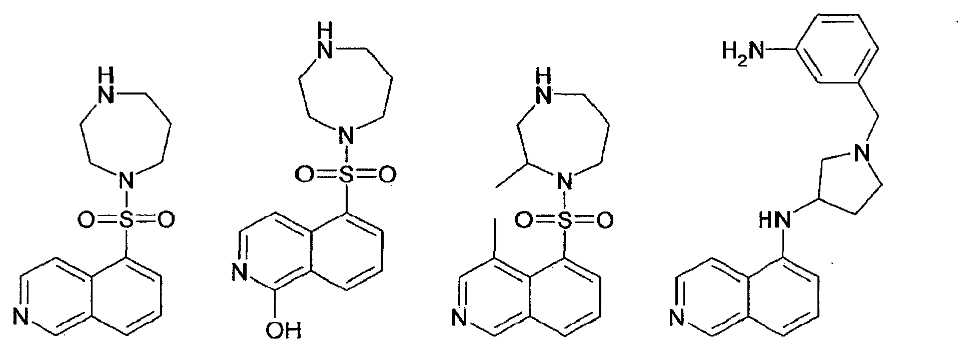 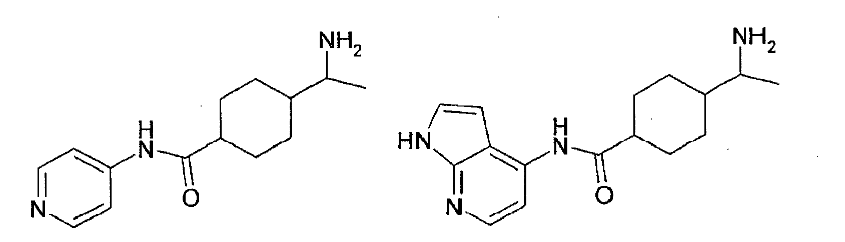 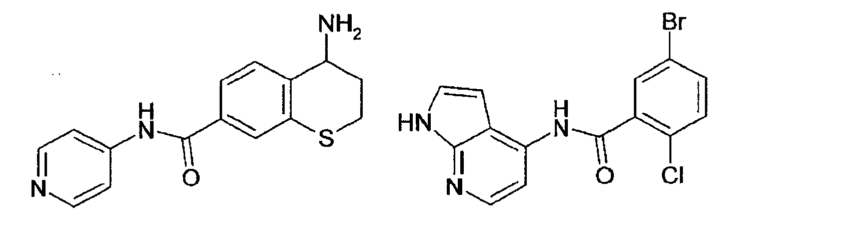 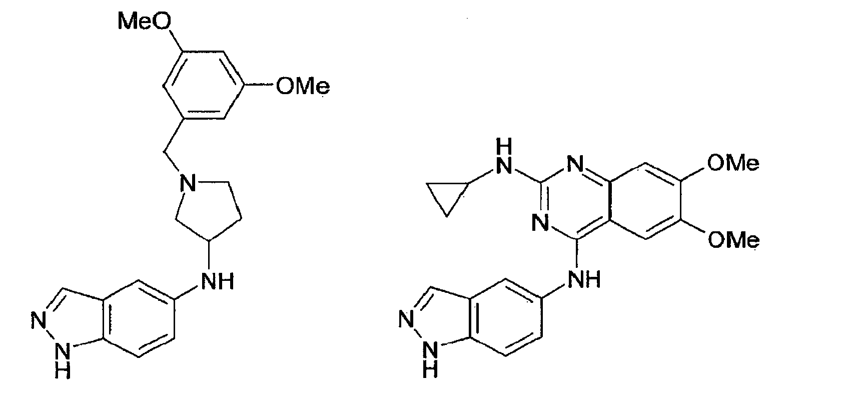 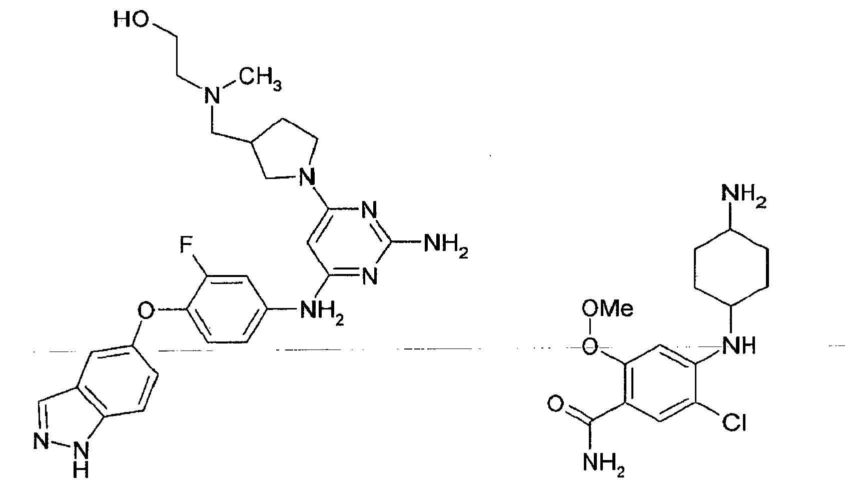 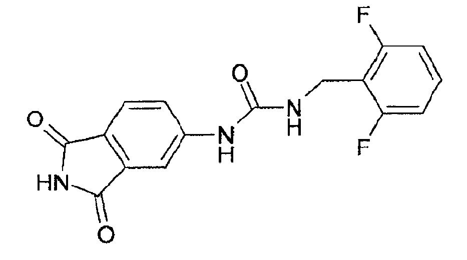 or pharmaceutically acceptable salts thereof.

6. The Rho kinase inhibitor for its use according to any of claims 1 to 5, which is selected from the group consisting of the compounds of the following formulae: 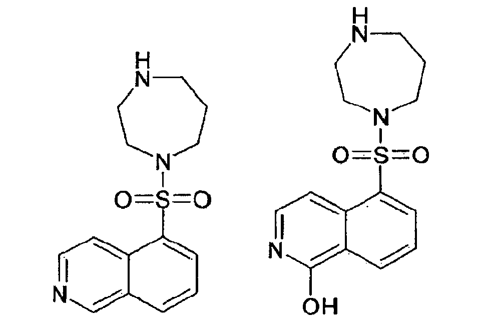 or pharmaceutically acceptable salts thereof.

7. The Rho kinase inhibitor for its use according to any of claims 1 to 6, wherein the medicament further comprises at least one compound having anti-parasitic activity.

8. The Rho kinase inhibitor for its use according to any of claims 1 to 7, wherein the compound having anti-parasitic activity has anti-malarial activity.

9. The Rho kinase inhibitor for its use according to claim 8, wherein the compound having anti-malarial activity is selected from the group consisting of Quinine, Quinidine, Quinine-doxycycline, Artemether, Artemotil, Artesunate, Arteether, Méfloquine, Amodiaquine, Dihydroartémisinine, Pipéraquine, Halofantrine, Atovaquone, Chloroquine, Dapsone, Doxycycline, a Cycline, Lumefanthrine, Proguanil, Pyrimethamine, Pyronaridine, Sulfadoxine, Diamidine, Ferroquine, Fluoroquinolone, Fosmidomycine, Tafenoquine, and Trioxaquine.

## Patentansprüche

1. Rho-Kinase-Inhibitor für die Verwendung als Arzneimittel in der Prävention und/oder der Behandlung von Krankheiten, die mit einer Infektion durch einen einzelligen Parasiten in Zusammenhang stehen, wobei der Rho-Kinase-Inhibitor die Rho-Kinase in der Zelle des Individuums, das gefährdet ist, infiziert zu werden oder durch einen einzelligen Parasiten infiziert ist, inhibiert.

2. Rho-Kinase-Inhibitor für dessen Verwendung gemäß Anspruch 1, wobei der einzellige Parasit aus der Gruppe bestehend aus Trypanosoma spp. und Plasmodium spp ausgewählt ist.

3. Rho-Kinase-Inhibitor für dessen Verwendung gemäß einem der Ansprüche 1 oder 2, wobei die Krankheiten aus der Gruppe bestehend aus Malaria und afrikanische Trypanosomiasis (Schlafkrankehit) ausgewählt sind.

4. Rho-Kinase-Inhibitor zu dessen Verwendung gemäß einem der Ansprüche 1 bis 3, wobei die Krankheiten aus der Gruppe bestehend aus schwerer Malaria, zerebrale Malaria, Multi-Organ-Versagen-Syndrom und fortgeschrittenes Stadium der afrikanischen Trypanosomiasis, ausgewählt sind.

5. Rho-Kinase-Inhibitor für dessen Verwendung gemäß einem der Ansprüche 1 bis 4, der aus der Gruppe bestehend aus den Verbindungen der folgenden Formeln ausgewählt ist: 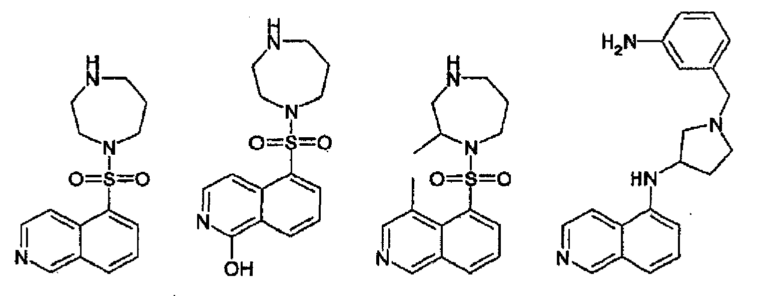 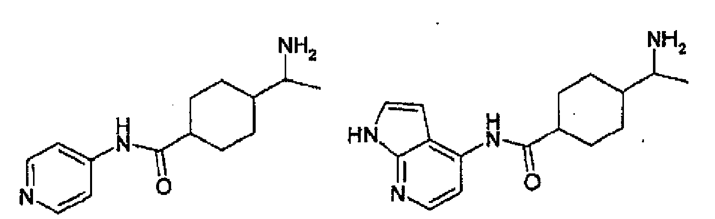 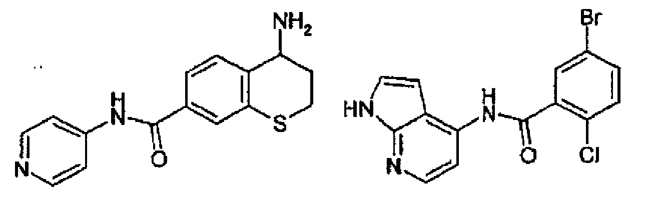 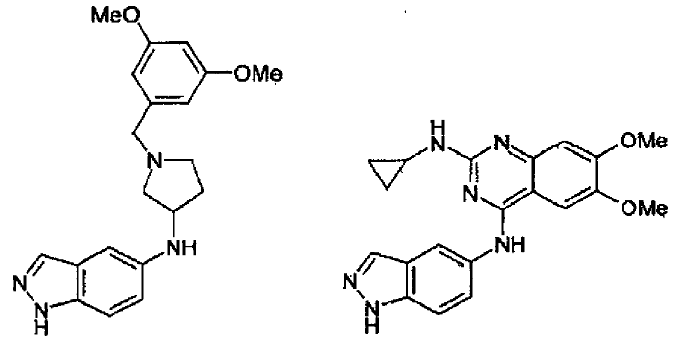 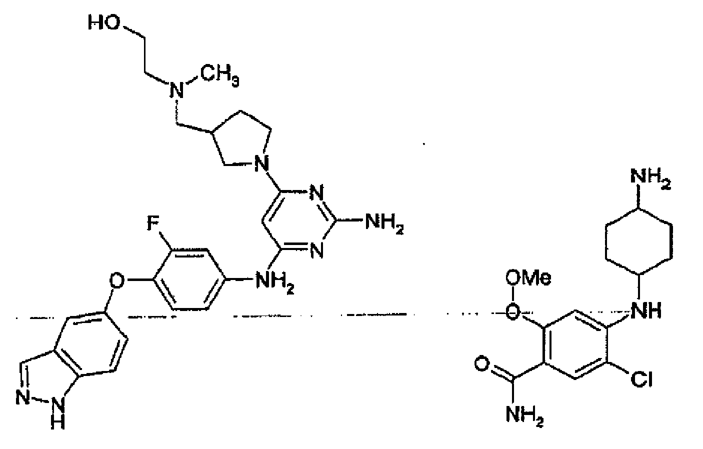 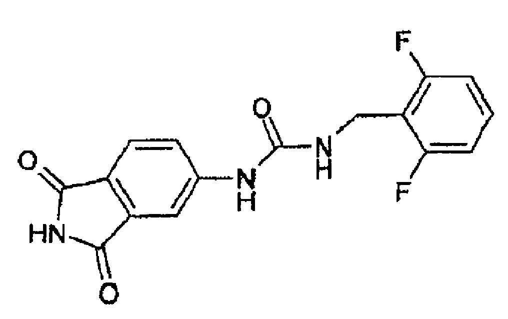 oder pharmazeutisch annehmbare Salze davon.

6. Rho-Kinase-Inhibitor für dessen Verwendung gemäß einem der Ansprüche 1 bis 5, der aus der Gruppe bestehend aus den Verbindungen der folgenden Formeln ausgewählt ist: 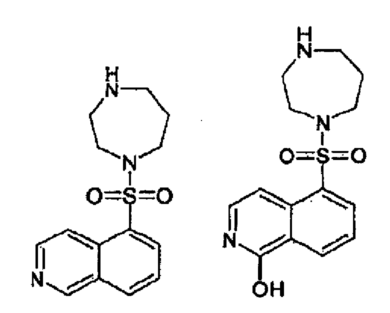 oder pharmazeutisch annehmbare Salze davon.

7. Rho-Kinase-Inhibitor für dessen Verwendung gemäß einem der Ansprüche 1 bis 6, wobei das Medikament zusätzlich mindestens eine Verbindung mit Anti-parasitärer-Aktivität enthält.

8. Rho-Kinase-Inhibitor für dessen Verwendung gemäß einem der Ansprüche 1 bis 7, wobei die Verbindung mit anti-parasitärer Aktivität Anti-Malaria-Aktivität aufweist.

9. Rho-Kinase-Inhibitor für dessen Verwendung gemäß Anspruch 8, wobei die Verbindung mit Anti-Malaria-Aktivität aus der Gruppe bestehend aus Quinin, Quinidin, Quinin-doxycylcin, Artemether, Artemotil, Artesunat, Arteether, Méfloquin, Amodiaquin, Dihydroartémisin, Pipéraquin, Halofantrin, Atovaquon, Chloroquin, Dapson, Doxycylcin, ein Cyclin, Lumefanthrin, Proguanil, Pyrimethamin, Sulfadoxin, Diamidin, Ferroquin, Fluoroquinolon, Fosmidomycin, Tafenoquin und Triooxaquin ausgewählt ist.

## Revendications

1. Inhibiteur de Rho kinase pour l'utilisation en tant que médicament dans la prévention et/ou le traitement de pathologies associées à une infection par un parasite protozoaire, dans lequel l'inhibiteur de Rho kinase inhibe la Rho kinase des cellules de l'individu qui présente un risque d'infection ou qui est infecté par un parasite protozoaire.

2. Inhibiteur de Rho kinase pour son utilisation selon la revendication 1, dans lequel le parasite protozoaire est choisi dans le groupe constitué des espèces *Trypanosoma spp.* et des espèces *Plasmodium spp.*

3. Inhibiteur de Rho kinase pour son utilisation selon la revendication 1 ou 2, dans lequel les pathologies sont choisies dans le groupe constitué de la malaria et de la trypanosomiase africaine (maladie du sommeil).

4. Inhibiteur de Rho kinase pour son utilisation selon l'une quelconque des revendications 1 à 3, dans lequel les pathologies sont choisies dans le groupe constitué de la malaria sévère, de la malaria cérébrale, du syndrome de défaillance polyviscérale et du stade avancé de la trypanosomiase africaine.

5. Inhibiteur de Rho kinase pour son utilisation selon l'une quelconque des revendications 1 à 4, qui est choisi dans le groupe constitué des composés répondant aux formules suivantes : 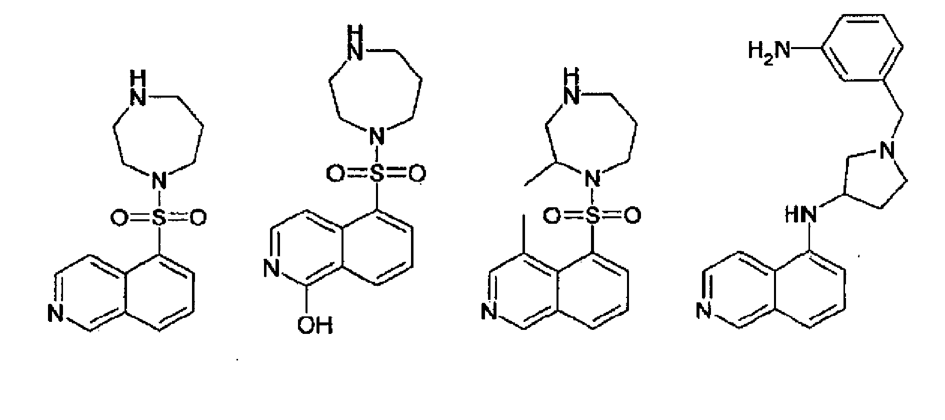 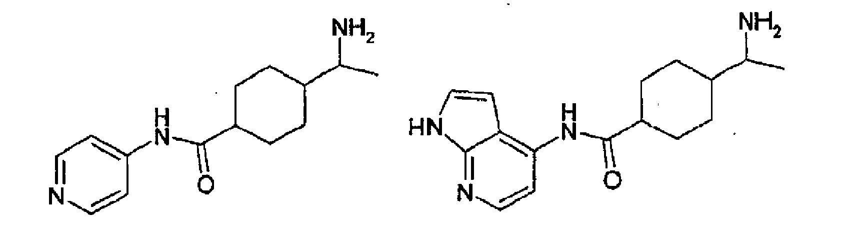 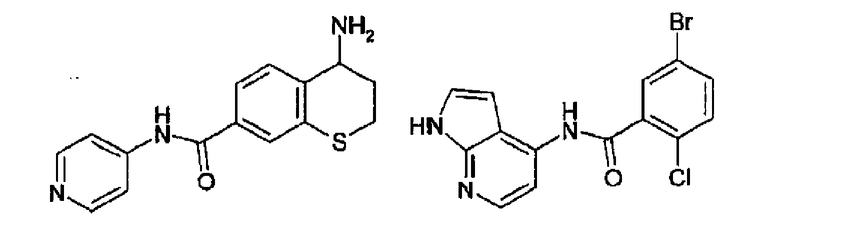 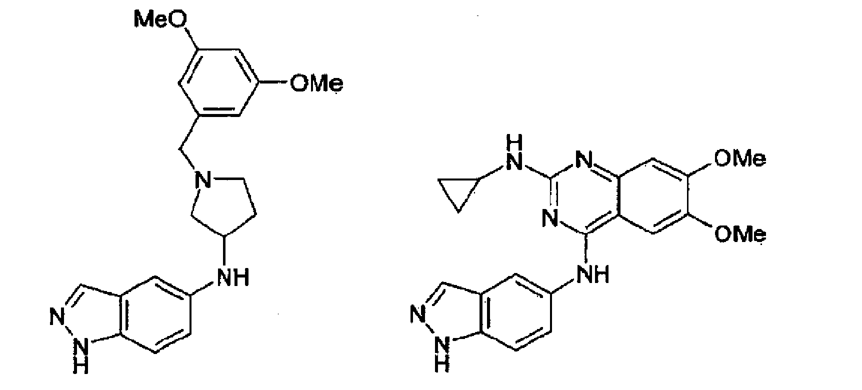 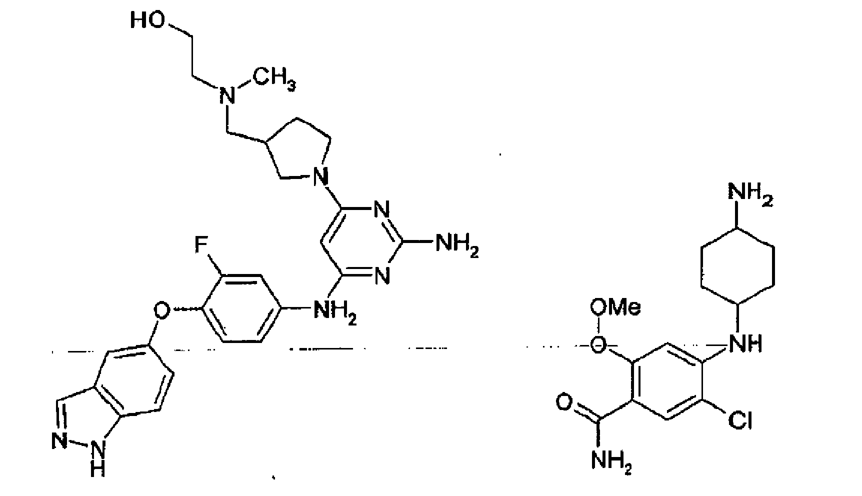 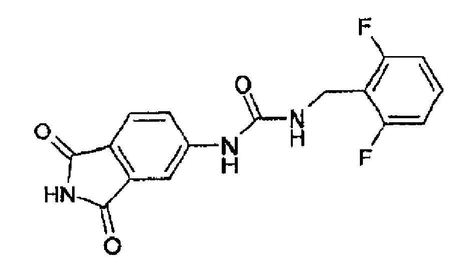 ou des sels pharmaceutiquement acceptables de celui-ci.

6. Inhibiteur de Rho kinase pour son utilisation selon l'une quelconque des revendications 1 à 5, qui est choisi dans le groupe constitué des composés répondant aux formules suivantes : 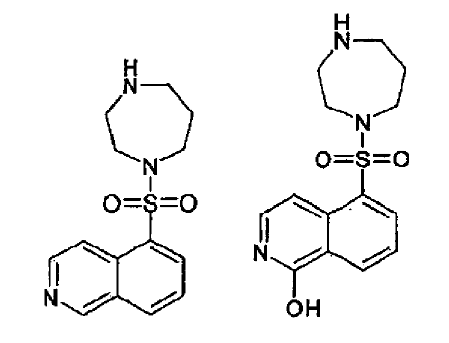 ou des sels pharmaceutiquement acceptables de celui-ci.

7. Inhibiteur de Rho kinase pour son utilisation selon l'une quelconque des revendications 1 à 6, dans lequel le médicament comprend en outre au moins un composé ayant une activité antiparasitaire.

8. Inhibiteur de Rho kinase pour son utilisation selon l'une quelconque des revendications 1 à 7, dans lequel le composé ayant une activité antiparasitaire a une activité anti-malaria.

9. Inhibiteur de Rho kinase pour son utilisation selon la revendication 8, dans lequel le composé ayant une activité anti-malaria est choisi dans le groupe constitué de la Quinine, de la Quinidine, de la Quinine-doxycycline, de l'Artéméther, de l'Artémotil, de l'Artésunate, de l'Artééther, de la Méfloquine, de l'Amodiaquine, de la Dihydroartémisinine, de la Pipéraquine, de l'Halofantrine, de l'Atovaquone, de la Chloroquine, de la Dapsone, de la Doxycycline, d'une Cycline, de la Luméfantrine, du Proguanil, de la Pyriméthamine, de la Pyronaridine, de la Sulfadoxine, de la Diamidine, de la Ferroquine, de la Fluoroquinolone, de la Fosmidomycine, de la Tafénoquine et de la Trioxaquine.
